# EUROPÄISCHE PATENTSCHRIFT

(11) **EP 2 662 106 B1**
(45) Veröffentlichungstag und Bekanntmachung des Hinweises auf die Patenterteilung: **20.04.2016**
(21) Anmeldenummer: 13002127.2
(22) Anmeldetag: 23.04.2013
(51) Int. Cl.: A61M 13/00

(54) **Insufflationsvorrichtung**
Insufflation device
Dispositif d'insufflation

(30) Priorität: 09.05.2012 DE 102012009078
(43) Veröffentlichungstag der Anmeldung: 13.11.2013
(73) Patentinhaber: Karl Storz GmbH & Co. KG, 78532 Tuttlingen (DE)
(72) Erfinder: Bardini, Romeo, I-35123 Padova (IT); Emmerich, Bernd, D-78532 Tuttlingen (DE); Krattiger, Beat, D-78532 Tuttlingen (DE)

(56) Entgegenhaltungen:
- WO-A2-2009/147638
- GB-A- 2 198 948
- US-A1- 2005 137 529
- US-A1- 2006 129 087
- US-A1- 2009 082 718
- US-A1- 2010 185 139

## Beschreibung

Die nachfolgende Erfindung bezieht sich auf eine Insufflationsvorrichtung, die in der Medizin- respektive Operationstechnik zugleich zur Insufflation und auch zur Gasdissektion geeignet ist, und auf ein entsprechendes Verfahren.

Die gleichzeitige Nutzung von Insufflatoren und Vorrichtungen zur Gasdissektion während Operationen im Bauchraum ist bekannt.

Die Insufflatoren dienen dabei dazu, dem Chirurgen in der endoskopischen Abdominalchirurgie durch ein Endoskop den Blick auf die zu operierende Stelle im Abdomen zu ermöglichen, indem Kohlendioxid (CO₂) in das Abdomen eingeleitet und damit die Bauchdecke angehoben wird. Über eine Kanüle - üblich ist im Stand der Technik die sogenannte Verres-Kanüle oder ein Trokar- wird dazu das Gas unter Druckprüfung in das Abdomen eingelassen, bis sich der gewünschte Innendruck an der bestimmten Stelle im Bauchraum eingestellt hat und ein Anheben der Bauchdecke herbeigeführt wird. Der so entstandene gasgefüllte Raum erlaubt nun einerseits die freie Sicht durch das Endoskop und schafft andererseits Platz für die Durchführung des chirurgischen Eingriffs, etwa einer Dissektion, also der Gewebetrennung mittels Gas.

Bekannte Insufflatoren umfassen im Wesentlichen ein Gehäuse, das über einen Anschluss an eine CO₂-Gas-Quelle (nachfolgend als CO₂-Quelle abgekürzt) und über einen entsprechenden Abgang verfügt, der mit einer Zuführleitung - etwa einem Silikonschlauch - mit der Kanüle bzw. dem Trokar verbindbar ist; ferner sind ein Anschluss an eine Stromquelle und Bedienelemente zur Betätigung der Vorrichtung vonnöten. Anschluss und Abgang sind gehäuseinnenseitig meist durch eine oder mehrere Verbindungsleitungen oder -kanäle miteinander fluidisch gekoppelt, wobei in der bzw. in den Kanälen Gasdruck-Prüfmittel bzw. Steuerungs- und Regelungsmittel, die entsprechende Ventile umfassen, vorliegen. Die entsprechenden Druckkontrollvorrichtungen im Insufflator überprüfen daher den Druck in der Bauchhöhle auch unter dem besonderen Aspekt, Verletzungen des Patienten zu vermeiden. Die bekannter Maßen angewendeten Drücke durch den Insufflator liegen daher bei etwa 0 bis 30 mm Quecksilbersäule, also bei bis zu 3990 Pa.

In bekannten Insufflatoren werden dabei hinsichtlich der pneumatischen Bauteile verschiedene Konstruktionsweisen realisiert. Die erste Bauweise sieht vor, dass die pneumatischen Komponenten am Gehäuse des Gerätes fest verschraubt sind und über Schlauchverbindungen fluidisch kommunizieren. Die Kommunikation der pneumatischen Bauteile mit der Elektronik (z. B. für Ventile und Drucksensoren) wird mittels Kabeln realisiert. Gemäß der zweiten Bauweise wird ein Pneumatikblock geschaffen, in dem alle pneumatischen Verbindungen untergebracht sind.

Eine weitere Konstruktionsweise ist in der DE 102009007393 A1 beschrieben.

Ein bekannter Insufflator ist etwa ein unter dem Markennamen CO₂-Endoflator® SCB erhältlicher Insufflator der Karl-Storz GmbH & Co. KG, Tuttlingen, Deutschland.

Die Dissektion hingegen verlangt den Einsatz einer Dissektionskanüle oder -spitze oder -sonde (hierin alternativ verstanden) zur Durchführung des endoskopischen oder auch laparoskopischen Verfahrens, dessen Ausführung durch die mittels Insufflation angehobene Bauchdecke erleichtert wird.

Eine für die Gasdissektion geeignete spezielle Gasdissektionssonde ist etwa als Komponente einer Gewebedissektionsvorrichtung und eines entsprechenden Dissektionsverfahrens in der WO 2009/147638 A2 beschrieben. Dieses Patent überwindet die Nachteile, die bis dorthin durch die allein mögliche mechanische oder thermomechanische Dissektion durch das mechanische Handhaben der Gewebelagen, der sogenannten Mobilisierung gegeben waren. Dabei wird durch eine manuelle Einstellvorrichtung ein Gasdruck, der vorzugsweise durch CO₂ bereitgestellt wird, von 0,02 bis 0,03 bar, also der 5 bis etwa 10-fache Druck des bei der Insufflation zur Bauchdeckenanhebung bereitgestellten Drucks, an der Dissektionssonde bereitgestellt, um die Gewebelagen zu trennen. Der Druck muss dabei während der Gewebetrennung konstant aufrecht gehalten bleiben. Um den Druck kontinuierlich auf dem gegebenen Wert, und das CO₂ bei kontinuierlicher Durchflussrate zu halten, wird eine zweistufige Druckminderung durch einen speziellen Insufflator vorgenommen, der daher als Dissektionsinsufflator ausgebildet ist. Durch die Dissektionssonde wird das Gas während der Operation mittels eines pneumatischen Manipulators zwischen die zu trennenden Gewebelagen eingetragen.

Aus der US2009/0082718A ist ein verzweigter Insufflationsschlauch mit einem umschaltbaren Ventil bekannt, der den Anschluss mehrerer laparoskopischer Kanülen für die Insufflation ermöglicht. Mit dieser Anordnung werden höhere Flussraten bei der Insufflation erreicht.

US2005/0137529A1 beschreibt ein System zur gleichzeitigen Gasinsufflation und Medikamentengabe.

US2006/0129087A1 beschreibt ein System zur gleichzeitigen Gasinsufflation in verschiedenen Körperhöhlen mit unterschiedlichen Drücken, wobei der Gasdruck von einem gemeinsamen Gerät reguliert wird.

Nachteilig ist bei der gleichzeitigen Ausführung von Insufflation zum Anheben der Bauchdecke und der davon abweichenden Insufflation mit erhöhtem Druck zu Dissektionszwecken, dass zwei Geräte zur Ausführung dieser beiden Verfahren im Operationssaal vorhanden sein müssen, die mit den entsprechenden Gasquellen verbunden sind und die die entsprechenden Gasdrücke für die unterschiedlichen Verfahren bereit stellen. Dies erfordert einerseits die Handhabung zweier lokal getrennter Geräte und ist andererseits wegen des erforderlichen Platzbedarfs nachteilig.

Ausgehend von diesem Stand der Technik liegt der vorliegenden Erfindung die Aufgabe zu Grunde, eine Vorrichtung zu schaffen, die es ermöglicht, mit verringertem apparativen Aufwand und zugleich die Insufflation zum Zwecke der Bauchdeckenanhebung und die insufflationsbasierte Dissektion auszuführen.

Diese Aufgabe wird durch eine Vorrichtung mit den Merkmalen des unabhängigen Anspruchs 1 gelöst.

Bevorzugte Weiterbildungen der Vorrichtung werden durch die Unteransprüche beschrieben.

Eine erste Ausführungsform der erfindungsgemäßen Insufflationsvorrichtung umfasst einen Insufflator, zumindest eine Gaszuführleitung, und zumindest eine Insufflationskanüle, die zum Insufflieren eines Insufflations-Gases über die Gaszuführleitung mit dem Insufflator verbunden ist. "Insufflieren" meint hierin das Einbringen von Gas in einen Bauchraum eines Menschen oder auch eines Tiers, um die Bauchdecke zum Zweck der besseren Zugänglichkeit einer zu untersuchenden oder zu operierenden Stelle im Abdomen anzuheben.

Der Insufflator umfasst ein Gehäuse mit einem Eingang und zumindest einem ersten Abgang für das Insufflations-Gas, an den Abgang ist eine Gaszuführleitung angeschlossen. "Gaszuführleitung" meint hierin eine Leitung, um das Gas von dem Gehäuse bis zu der Insufflationskanüle zu transportieren. Die Leitung kann durch einen Silikonschlauch gebildet werden, der gegen das entsprechende Gas - das vorzugsweise CO₂ sein kann, insoweit inert ist, als das Gas nicht durch das Schlauchmaterial entweichen oder mit diesem eine chemische Reaktion eingehen kann. In dem Gehäuse liegt zwischen dem Eingang und dem ersten Abgang ein "Fluidpfad" oder "Fluidpfade" vor, in den die eine Mess-, Steuerungs- und Regelungsvorrichtung zum gesteuerten und geregelten Ausgeben des Gases zwischengeschaltet ist, die elektronisch zumindest mit Bedienmitteln verbunden ist. Diese können, müssen aber nicht an dem Gehäuse vorliegen. "Fluidpfad" bedeutet, dass ein definierter Weg für das Gas innerhalb des Gehäuses bereitgestellt ist, der durch Schlauchverbindungen, Rohre, aber auch durch Kanäle, die in eine oder zwischen zwei Platten eingebracht sind, gebildet werden. Derartige Mess-, Steuerungs- und Regelungsvorrichtungen in Insufflatoren sind dem Fachmann bekannt; sie können Flow-Meter, Ventile und weitere Komponenten umfassen, um den Gasstrom mit dem gewünschten und für die Anwendung geeigneten vorbestimmten Durchfluss und auf den gewünschten Druck einzustellen. Dabei ist es auch grundsätzlich bekannt, vorzusehen, dass ein Rückstrom von Gas aus dem Bauchraum in die Mess-, Steuerungs- und Regelungsvorrichtung geführt wird, um den dort vorliegenden Druck zu prüfen und um in Abhängigkeit des vorliegenden Druckes die Gaszufuhr zu steuern und zu regeln.

Erfindungsgemäß verfügt nun der Insufflator über eine Gaszuführleitung die zusätzlich mit einer pneumatischen Gewebedissektionssonde fluidisch verbunden ist, die ebenfalls elektronisch mit der Mess-, Steuerungs- und Regelungsvorrichtung gekoppelt ist. Die Insufflationsvorrichtung hat daher eine Umschaltvorrichtung, die dazu ausgebildet ist, eine Umschaltung des geregelten und gesteuerten Gasflusses zur Gasausgabe entweder aus der Gewebedissektionssonde oder aus der zumindest einen Insufflationskanüle bereitzustellen.

Die Gaszuführleitung, die die Gewebedissektionssonde mit Gas versorgt, kann dabei grundsätzlich dieselbe sein, wie die Gaszuführleitung, die die Insufflationskanüle mit Gas versorgt. Die Insufflationskanüle kann zum Beispiel eine Bardini-Kanüle sein, wie sie in der WO2009/147638 offenbart ist; es kann aber auch eine Ausführung mit getrennten Gaszuführleitungen vorgesehen sein. Entsprechend der Ausgestaltung kann dann die Umschaltvorrichtung das Umschalten stromaufwärts der Gaszuführleitungen oder darin vorsehen.

Vorteilhaft kann so ein an sich bekannter Insufflator durch Vorsehen der Umschaltvorrichtung und entsprechender Ausbildung der Mess-, Steuerungs- und Regelungsvorrichtung sowie der nötigen Anschlüsse für eine oder mehrere Gaszuführleitungen und elektronische Verbindungen genutzt werden, um im Insufflationsmodus, also zum Gasinsufflieren zum Zwecke der temporären Herstellung einer Höhle im Abdomen und alternierend dazu im (pneumatischen) Dissektionsmodus, also zum Zwecke der Trennung von Gewebelagen mittels Gas, genutzt zu werden. Dabei entfällt die Notwendigkeit, einen weiteren Insufflator, der nur im Dissektionsmodus arbeitet, bereitzustellen; es wird Platz im OP eingespart und das Ausführen zweier für die Operation notwendiger Verfahren lässt sich vereinfacht mit einer einzigen Vorrichtung ausführen.

Die Umschaltvorrichtung umfasst Umschaltmittel, die elektronisch mit der Mess-, Steuerungs- und Regelungsvorrichtung gekoppelt oder die in die Mess-, Steuerungs- und Regelungsvorrichtung integriert sind.

Die Umschaltvorrichtung zur Gasausgabe aus der Gewebedissektionssonde oder alternativ aus der Insufflationskanüle kann in einer Ausführungsform der erfindungsgemäßen Insufflationsvorrichtung umfassen, dass die Gaszuführleitung als eine Y-Gaszuführleitung ausgebildet ist. Dabei ist ein Abschnitt mit dem ersten Abgang für Gas am Gehäuse verbunden, der quasi ein Stück gemeinsamer Gaszuführleitung sowohl für den Dissektionsmodus als auch für den Insufflationsmodus bildet, und der sich dann verzweigt.

An der Verzweigung teilt sich die Gaszuführleitung in einen ersten distalen Abschnitt, der mit der Insufflationskanüle verbunden ist, und in einen zweiten distalen Abschnitt, der mit der Gewebedissektionssonde verbunden ist. Dabei sind die Umschaltmittel an der Verzweigung der Y-Gaszuführleitung vorgesehen. Derartige geeignete Umschalt- oder Umlenkmittel für Gas sind dem Fachmann an sich bekannt; es kann sich etwa um eine Weiche oder ein Umschaltventil handeln, die/das so ausgebildet ist, dass die Gaszuführung von dem gemeinsamen Abschnitt dann nur noch in einen der beiden distalen Abschnitte erfolgt.

Alternativ kann vorgesehen sein, dass statt der Umschalt- oder Umlenkmittel an der Verzweigung der vorstehenden Y-Gaszuführleitung die Umschaltvorrichtung durch jeweils eine mechanisch oder elektronisch betätigbare Gasabsperrvorrichtung in den distalen Abschnitten gebildet wird. Damit wird ein Gasausgang an der Insufflationskanüle oder alternativ der Dissektionssonde verschlossen und es ist nur noch das Abfließen des Gases durch den entsprechend geöffneten Gasausgang möglich. Vorzugsweise ist die Gasabsperrvorrichtung, die die Gaszuführung von dem Abschnitt in jeweils einen der distalen Abschnitte zulässt, über eine elektronische Kommunikationsverbindung mit der Mess-, Steuerungs- und Regelungsvorrichtung operativ verbunden, um zu gewährleisten, dass die Parameter zum Betreiben des jeweiligen der beiden Operationsmodi zeitgleich mit dem Verschließen der einen Gasabsperrvorrichtung und mit dem Öffnen der anderen korrekt eingestellt werden - insbesondere werden Gasdruck und Durchflussrate auf den entsprechenden Modus angepasst.

Die Weiche bzw. das Umschaltventil an der Y-Gaszuführleitung kann mechanische oder elektronische Betätigungsmittel aufweisen. Es ist dem Fachmann bekannt, dass sämtliche elektronischen Verbindungen in der vorliegenden Erfindung bzw. ihren Ausführungsformen geeignet mit Kabel oder ohne Kabel, also kabellos geführt werden können; dann sind Signalübertragungsvorrichtungen an den elektronisch kommunizierenden Partnern/ Vorrichtungen vorzusehen.

Eine Alternative zu der vorstehenden Ausführung mit der Y-Gaszuführleitung besteht darin, dass zur Gasausgabe entweder aus der Gewebedissektionssonde oder aus der zumindest einen Insufflationskanüle zwei Gaszuführleitungen bereitgestellt sind. In diesem Falle wird erfindungsgemäß vorgeschlagen, dass die Umschaltmittel im Gehäuse des Insufflators durch eine Umschalteinheit gebildet werden, die folglich den Gasstrom stromaufwärts der Gasabgänge am oder im Gehäuse umlenkt. In dieser Ausführungsform ist eine erste Gaszuführleitung einenends mit der Insufflationskanüle und andernends über den ersten Abgang und einen ersten Fluidpfad mit der Umschalteinheit verbunden und ein zweite Gaszuführleitung einenends mit der Gewebedissektionssonde und anderenends über einen zweiten Abgang, der an dem Gehäuse angeordnet ist, und über einen zweiten Fluidpfad mit der Umschalteinheit verbunden.

Die Umschalteinheit für die vorstehende Ausführungsform sieht zumindest eine Weiche oder ein Umschaltventil vor, um die Gaszuführung in jeweils einen der Fluidpfade sicher zu stellen, die in die beiden Gaszuführleitungen zur Versorgung der Dissektionssonde oder alternativ der Insufflationskanüle münden; oder die Umschalteinheit kann jeweils eine jedem der Fluidpfade zugeordnete mechanisch oder elektronisch betätigbare Gasabsperrvorrichtung aufweisen.

Es ist zu erwähnen, dass es grundsätzlich möglich ist, das vorliegende Prinzip nach beiden Alternativen auch so auszubilden, dass zwei oder mehr Insufflationssonden, die auch in Trokare integriert sein können, durch den Insufflator mit Gas beschickt werden.

Darüber hinaus kann für sämtliche der vorstehenden Ausführungsbeispiele vorgesehen sein, dass die Gewebedissektionssonde einen Schalter aufweist, der elektronisch direkt oder über die Mess-, Steuerungs- und Regelungsvorrichtung mit den Umschaltmitteln, die in der jeweiligen Ausführungsform der Insufflationsvorrichtung vorliegen, operativ verbunden ist. Der Schalter kann also mit der Weiche an der Y-Verzweigung ebenso verbunden sein, wie mit der Umschaltvorrichtung in der Zwei-Gaszuführleitungs-Variante.

Betätigen des Schalters bewirkt, dass etwa vom Insufflationsmodus auf den DissektionsModus umgeschaltet wird, der sich insbesondere dadurch auszeichnet, dass der letzte Gas-Druckwert des Insufflationsmodus' bei Gas-Flow als Referenzwert, ggf. auch der Durchschnittswert einer definierten davor liegenden Zeit, berücksichtigt wird, und bei einer Aktivierung durch Betätigen des Schalters an der Dissektionssonde bzw. deren Kanüle durch einen Operateur wird ein um einen definierten Wert höherer Gas-Druck über den Weg, der zur Versorgung der Dissektionssonde zur Verfügung steht, über die Kanüle hinaus in eine Patientenkavität hinein gelenkt. Die Gaszufuhr über die Dissektionssonde erfolgt so lange, bis der Operateur den Schalter loslässt oder betätigt, um den Dissektionsmodus zu beenden oder zu unterbrechen.

Alternativ zu dem Betätigungsschalter an der Dissektionssonde, oder zusätzlich, kann die Insufflationsvorrichtung ein Pedal aufweisen, das elektronisch direkt oder über die Mess-, Steuerungs- und Regelungsvorrichtung mit den Umschaltmitteln operativ verbunden ist. Hierin umfasst "Umschaltmittel" wieder sämtliche Vorrichtungen von der Umschalteinheit in der Zwei-Gaszuführleitungs-Variante, bis hin zu der Weiche, respektive dem Ventil an der Y-Verzweigung oder Gasabsperrvorrichtungen in oder an distalen Abschnitten der Y-Gaszuführleitung.

Außerdem kann die Dissektionssonde vorteilhafterweise mit Mitteln zur Identifizierung der Dissektionssonde ausgestattet sein. Wenn die Dissektionssonde mit der Gaszuführleitung bzw. mit elektrischen Leitungen verbunden wird, kann vorgesehen sein, über diese Mittel und die vorhandenen Leitungen dem Insufflator mitzuteilen, dass die Dissektionssonde angeschlossen ist und um welche Art von Dissektionssonde es sich handelt. Abhängig von dieser Information können dann beispielsweise die zuvor beschriebenen Umschaltmittel angesteuert werden, um die Fluidverbindung mit der Dissektionssonde herzustellen. Auch kann der Insufflationsmodus des Insufflators bei Anschluss und Erkennung der Dissektionssonde so automatisch ausgelöst werden. Je nach Dissektionssonde können verschiedene Modi mit verschiedenen Parametern für Druck und Flow vorgesehen sein, die dem Insufflator bei der Erkennung mitgeteilt werden.

Dies hat den Vorteil, dass die Bedienung erleichtert wird und Parameter nicht von Hand eingestellt werden müssen, was auch der Sicherheit dient.

Die Mittel zur Identifizierung können beispielsweise elektronische Speicherbausteine sein, in denen die Information über die Dissektionssonde hinterlegt wird. Über eine entsprechende Erkennungseinrichtung, die entweder direkt am Insufflator vorgesehen ist oder auch über elektrische Leitungen mit diesem verbunden ist, kann die Information aus der Dissektionssonde ausgelesen werden.

Alternativ kann die Identifizierung über Dioden, Widerstände oder sogenannte Radio Frequency Identification Systeme (RFID) erfolgen, deren Verwendung dem Fachmann bekannt ist. Auch kann das Instrument mit einem Strichcode, wie einem Barcode oder Matrixcode, zur Identifizierung versehen sein. Am Insufflator selbst oder einer damit verbundenen separaten Einrichtung wird der Strichcode hierfür ausgelesen.

Die Insufflationskanüle, von der hierin die Rede ist, kann in einem Trokar umfasst sein, und das Gas wird in aller Regel CO₂-Gas sein.

Ferner ist es möglich, dass zumindest eine der Gaszuführleitungen, die mit der Insufflationskanüle und/oder mit der pneumatischen Gewebedissektionssonde verbunden ist/sind, doppellumig ausgebildet ist oder sind. Dabei

dient ein erstes Lumen zur Zuführung des Gases in die Insufflationskanüle oder die Gewebedissektionssonde, und ein zweites Lumen zur Rückführung zumindest eines Teils des Gases in die Mess-, Steuerungs- und Regelungsvorrichtung in den Insufflator. Damit kann der Gasdruck, der in der Körper- oder Bauchhöhle herrscht gemessen und geprüft und als Steuerungs- und Regelungsparameter durch die Steuerungs- und Regelungsvorrichtung genutzt werden. So kann gezielt eine Druckreduzierung bei manueller Dissektions-Modus-Aktivierung im Gasinsufflationsgerät selbst herbeigeführt werden.

Alternativ zu der doppellumigen Ausführung wird vorgeschlagen, dass zumindest eine Gasrückführleitung bereitgestellt wird, die nicht die mit der Insufflationskanüle und/oder mit der pneumatischen Gewebedissektionssonde verbundene Gaszuführleitung ist/sind. Diese zusätzliche Leitung dient zur Rückführung zumindest eines Teils des Gases von der Insufflationskanüle oder einem weiteren Trokar über einen entsprechenden zusätzlichen Anschluss in die Mess-, Steuerungs- und Regelungsvorrichtung in den Insufflator. Sie ist ausgebildet zum vorstehenden Zweck, nämlich zur Überprüfung des Gasdrucks in der Bauchhöhle.

Die elektronischen Verbindungen, die zum Betreiben der Insufflationsvorrichtung vorgesehen sind, können jeweils direkt durch eine elektrische Leitung und einen entsprechenden Anschluss, oder über eine Signalübertragungsvorrichtung, die eine Sender-Empfängervorrichtung zur Übermittlung eines elektronischen Signals umfasst, hergestellt werden. Werden Leitungen verwendet, so können diese parallel mit den Gaszuführleitungen und in einer gemeinsamen Ummantelung geführt werden. Vorteilhafterweise werden Kombinationsleitungen verwendet in Form von Gaszuführleitungen in deren Wandung oder Ummantelung die elektrischen Leitungen untergebracht sind. Es kann sich um Metallleiter, z.B, Drähte, oder um Leitungen aus leitfähigem Kunststoff handeln, die von dem isolierenden Kunststoff der Schläuche der Gaszuführleitungen umgeben sind. So können auf einfache Weise und platzsparend Fluid einerseits und Strom und elektrische Signale andererseits in einer gemeinsamen Leitung geführt werden.

Die elektronischen Verbindungen liegen zum Beispiel zwischen dem Schalter, der an der Gewebedissektionssonde angeordnet ist, und/oder den Gasabsperrvorrichtungen, und/oder den Umschaltmitteln an der Verzweigung und/oder dem Pedal sowohl untereinander vor, als auch (oder nur) zwischen diesen Vorrichtungen und der Mess-, Steuerungs- und Regelungsvorrichtung.

Alternativ kann die elektronische Kommunikation über eine Kommunikations-BUS-Verbindung erfolgen, die mit dem Insufflator hergestellt wird. Der Kommunikationsbus kann in einem separaten Gerät bereitgestellt werden.

Ferner kann erfindungsgemäß vorgesehen sein, dass die Aktivierungsdauer der Gewebedissektionssonde vorbestimmbar ist und mittels eines Timers, der mit der Mess-, Steuerungs- und Regelungsvorrichtung operativ gekoppelt ist, voreingestellt werden kann. Ergänzend kann auch eingestellt werden, dass die Aktivierungsdauer einen vorgegebenen Grenzwert aus Sicherheitsgründen nicht überschreitet. Selbiges gilt für die Vorgabe eines maximal zulässigen Insufflations- bzw. Dissektionsdrucks, respektive des Drucks, der für den jeweiligen Modus maximal als zulässig vorgegeben wird.

Die Einstellung der vorgenannten Grenzwerte, aber auch von Parametern, die den einzelnen Modi bspw. für die Ausführung bestimmter Operationsarten zugeordnet werden sollen, kann entweder über die Bedieneinrichtung am Gehäuse vorgenommen werden, oder es kann bereits Herstellerseitig oder im Nachgang die Einstellung vorgenommen werden. Ggfs. über eine Programmiervorrichtung, die kommunikativ mit der Mess- Steuerungs- und Regelungsvorrichtung verbunden wird, die geeigneter Weise eine Speicher- und gegebenenfalls eine Recheneinheit umfasst.

Ebenfalls aus Sicherheitsgründen kann die Insufflationsvorrichtung eine Signalvorrichtung zur Ausgabe eines akustischen und/oder optischen Warnsignals aufweisen. Durch diese Signalvorrichtung, die eingestellt bzw. aktiviert werden kann, kann ein Warnsignal bei Überschreiten eines vorbestimmten Insufflations- oder Dissektionsdrucks, einer vorbestimmten Gasdurchflussrate des Gases, das in die Gaszuführleitung zugeführt wird, oder der Aktivierungsdauer der Gewebedissektionssonde ausgegeben werden. Dies umfasst, dass auch vorgesehen bzw. eingestellt werden kann, dass ein Alarm, der bei Überschreiten des vorbestimmten Insufflationsdrucks ("Normaldruck") erfolgen würde, der aber dem gewünschten Dissektionsdruck entspricht, unterdrückt bzw. nicht ausgelöst wird, wenn das Gerät im Dissektionsmodus betrieben wird und dazu einen höheren Innendruck (in der Körperhöhle) erfordert, als er bei Vorliegen eines Normaldrucks ist, da zur Gewebedissektion der höhere Druck notwendig ist.

Ferner kann auch durch Erzeugung einer "künstlichen Leckage" in dem System, das zur Insufflation erforderlich ist, also in den Fluidpfaden und Gaszuführ- bzw. - rückführleitungen, die das Insufflationssystem bilden, ein zu hoher Druck abgelassen werden. Dies kann auch durch einen an das Insufflationssystem angeschlossenen weiteren anderen Trokar erfolgen. Mehrere Trokare liegen üblicherweise vor, wenn das Gerät zur Laparoskopie genutzt wird. Alternativ kann auch bei NOTES (Natural Orifice Translumenal Endoscopic Surgery) über eine separate flexible Gaszuführleitung mit Kanüle über einen zweiten, im flexiblen Instrument befindlichen Kanal Gas kontrolliert abgelassen werden. Diese Maßnahme reduziert den notwendigen Druck zur Dissektion, da dieser Dissektionsdruck immer in einem geschlossenen System gegen den Innendruck im bspw. Abdomen entgegenwirken muss, um eine Aufweitung/ Dissektion zu erwirken. Die Ablasseinrichtung ist mit der Mess-, Steuerungs- und Regelungsvorrichtung elektronisch gekoppelt, so dass die Absperreinrichtung immer dann geöffnet wird, wenn sich aus der Betätigung einer entsprechenden Komponente der Umschaltvorrichtung ergibt, dass der Dissektionsmodus ausgelöst wird. Das Ablassen von Gas könnte auch automatisiert erfolgen, wenn, wie oben dargelegt, ein entsprechender unerwünschter Überdruck erkannt wird.

Mit der Vorrichtung kann erfindungsgemäß ein Verfahren zum alternierenden Insufflieren von Gas in eine Körperhöhlung und pneumatischem Dissezieren in derselben unter Verwendung einer Ausführungsform der erfindungemäßen Insufflationsvorrichtung ausgeführt werden. Das Verfahren, umfasst im Wesentlichen die Schritte
a) Verbinden des Eingangs (15) des Insufflators (10) mit einer Gasquelle und Zuführen von Gas in den Fluidpfad, in den eine Mess-, Steuerungs- und Regelungsvorrichtung (17) zum gesteuerten und geregelten Ausgeben des Gases zwischengeschaltet ist,
b) Aktivieren der Bedienmittel (14) und über die Gaszuführleitung und die Insufflationskanüle (30) Zuführen von Gas in den Bauchraum gemäß einem Insufflationsmodus,
c) Betätigen der Umschaltvorrichtung und Umlenken des geregelten und gesteuerten Gasflusses von der Gasausgabe aus der Insufflationskanüle (30) zu der Gasausgabe aus der Gewebedissektionssonde (20) gemäß einem Dissektionsmodus,
d) Betätigen der Umschaltvorrichtung und Umlenken des geregelten und gesteuerten Gasflusses von der Gasausgabe aus der Gewebedissektionssonde (20) zu der Gasausgabe aus der Insufflationskanüle (30),
e) in einem vorbestimmten Umfang Wiederholen der Schritte c) und d), danach
f) Beenden der Gaszufuhr.

Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich eine schematische Darstellung von Ausführungsbeispielen der Erfindung.
- **Fig. 1**: zeigt eine schematische Ansicht einer Insufflationsvorrichtung gemäß dem Stand der Technik,
- **Fig. 2a**: zeigt eine schematische Ansicht einer erfindungsgemäßen Insufflationsvorrichtung,
- **Fig. 2b**: zeigt ein Detail A einer erfindungsgemäßen Insufflationsvorrichtung
- **Fig. 3a**: zeigt eine schematische Skizze einer Insufflationsvorrichtung mit Y-Gaszuführleitung,
- **Fig. 3b**: zeigt eine schematische Skizze Insufflationsvorrichtung mit zwei Gaszuführleitungen.

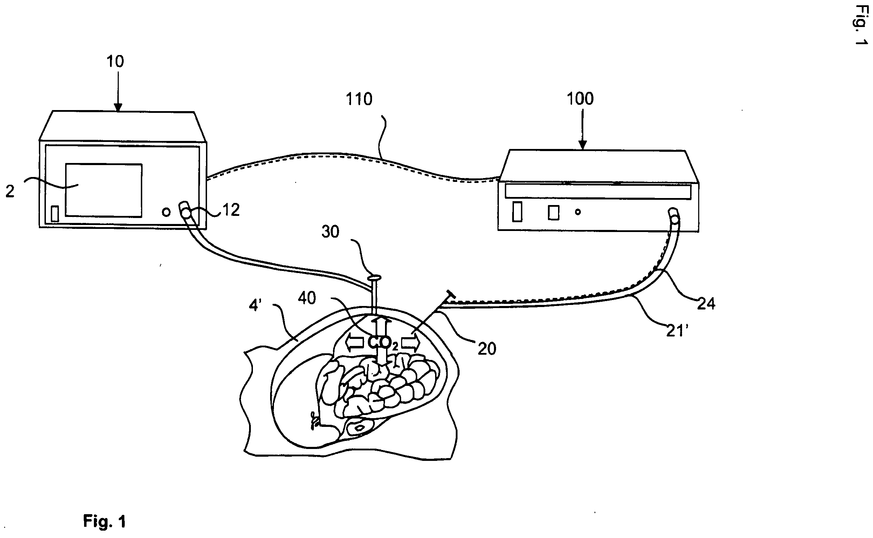

Die Ausführungsformen der vorliegenden erfindungsgemäßen Insufflationsvorrichtung dienen dazu, mit einem einzigen Gerät während eines operativen Eingriffs eines Menschen oder eines Tiers, der es erfordert, dass eine Körperhöhle wie die Bauchhöhle durch Gaseinführen ausgedehnt wird, um darin auf verbesserte Weise untersuchen oder operieren zu können, zugleich eine pneumatische Dissektion ausführen zu können. Im Stand der Technik, siehe beispielhaft Fig. 1, ist es bis dato erforderlich, dass über einen Insufflator 10 eine Insufflationskanüle, dort ein Trokar 30, über den Anschluss 12 geregelt und gesteuert mit CO₂ versorgt wird. Der Anschluss an die CO₂-Quelle ist nicht in der Fig. 1 gezeigt. Wie zu sehen ist, ragt der Trokar 30 in die Bauchhöhle 40 eines Menschen und bläst CO₂ ein (siehe kleine Blockpfeile). Die Bauchdecke 4' hebt sich, der Arbeitsraum im Abdomen 40 wird vergrößert. Eine Dissektionssonde 20 wird parallel an einen zweiten Insufflator 100 angeschlossen. Über eine BUS-Kommunikationsverbindung stehen die beiden Insufflatoren 10,100 in kommunikativer Verbindung. Wie zu sehen ist, ist die Dissektionssonde 20 bzw. deren Betätigungsschalter fluidisch über die Leitung 21' und elektronisch über eine Leitung 24 mit dem Insufflator 100 verbunden. An dem Insufflator 10 ist ein Display 2 zu sehen, das beispielsweise den Gasdruck in der Bauchhöhle 40 und die CO₂-Durchflussrate anzeigt, die durch eine Mess-, Steuerungs- und Regelungsvorrichtung gemessen und kontrolliert wird. Dort sind zwei Insufflatoren 10,100 nötig, die es ermöglichen, dass das Insufflieren und Dissezieren ausgeführt werden kann.

Erfindungsgemäß wird nun vorgeschlagen, dass eine Insufflationsvorrichtung genutzt wird, die nur einen einzigen Insufflator 10 einsetzt.

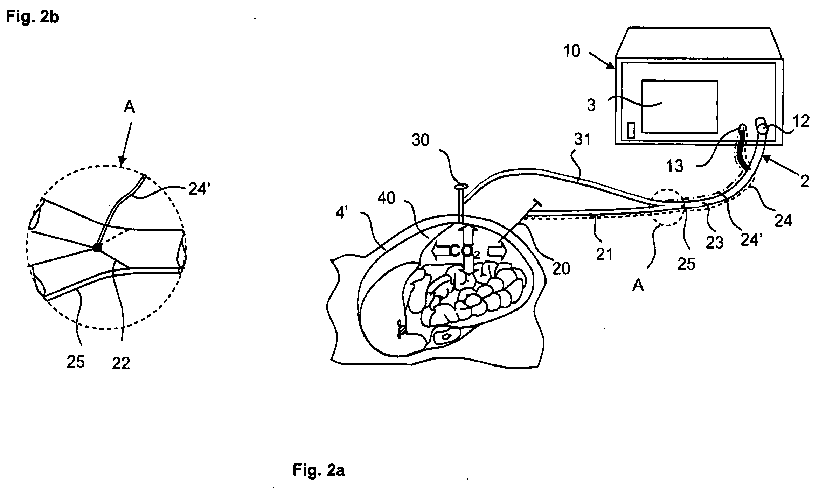

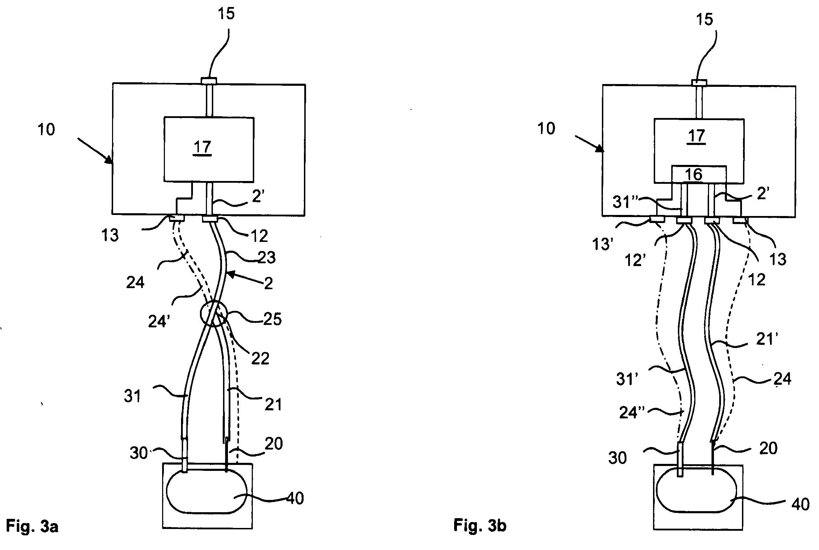

Ein solcher erfindungsgemäßer Insufflator 10 verfügt, wie Fig. 3a und 2a zeigen, grundsätzlich über wenigstens eine Gaszuführleitung 2 und zumindest über eine Insufflationskanüle 30, die zum Insufflieren eines Insufflations-Gases über die Gaszuführleitung 2 mit dem Insufflator 10 verbunden ist. Dieser weist ein Gehäuse mit einem Eingang 15 auf, der mit einer CO₂-Quelle verbindbar ist. In dem Gehäuse liegt zwischen dem Eingang 15 und dem ersten Abgang 12 ein Fluidpfad (Fig. 3a) vor, in den eine Mess-, Steuerungs- und Regelungsvorrichtung 17 zum gesteuerten und geregelten Ausgeben des Gases zwischengeschaltet ist, die elektronisch mit an dem Gehäuse vorliegenden Bedienmitteln 3 verbunden ist. Die Bedienmittel können, siehe Fig. 2a als Touchscreen 3 ausgebildet sein und zugleich der Anzeige und der Betätigung dienen. Natürlich können die Bedien- und Anzeigemittel 3 auf verschiedene andere, dem Fachmann bekannte Weise ausgestaltet sein.

Weiter ist der Insufflator 10 mit einer pneumatischen Gewebedissektionssonde 20 fluidisch verbunden, die elektronisch mit der Mess-, Steuerungs- und Regelungsvorrichtung 17 verbunden ist. Die Insufflationsvorrichtung verfügt erfindungsgemäß über eine Umschaltvorrichtung, die bewirkt, dass der Gasfluss auf geregelte und gesteuerte Weise entweder durch die Gewebedissektionssonde 20 oder durch die Insufflationskanüle 30 strömt.

Das erfindungsgemäße Prinzip lässt sich dabei im Wesentlichen auf zwei Weisen realisieren, wobei die erste im Wesentlichen durch Figuren 2a, 2b und 3a und die zweite Weise durch Fig. 3b veranschaulicht wird:
- es gibt nach Weise 1 nur einen Gasausgang 12, und der Gasfluss wird von einer gemeinsamen Gaszuführleitung 2 an einer Verzweigung 25 mittels Umschaltmitteln in die Gewebedissektionssonde(n) 20 oder alternativ die Insufflationskanüle(n) 30 gelenkt, oder
- es gibt nach Weise 2 zwei Typen von Gaszuführleitungen 21',31', und die Umschaltmittel liegen bereits stromaufwärts dieser Gaszuführleitungen 21',31' vor, um das Gas auf die gewünschte Weise zu lenken.

Dabei ist - was hierin nicht besonders ausgeführt wird, da das Wirkprinzip bekannt ist - auch wenigstens eine Leitung vorzusehen, mittels der bei beiden Weisen Gas aus der Körperhöhle in den Insufflator 10 bzw. die Mess-, Steuerungs- und Regelungsvorrichtung 17 rückgeführt wird, um den dort vorliegenden Druck zu prüfen und die Sicherheit des Patienten auch hinsichtlich Verletzungen durch zu hohen Druck zu kontrollieren. Weiter können in beiden Weisen Vorrichtungen zum Ablassen von Gas vorgesehen sein. Schließlich ist es auch in beiden Weisen möglich, dass die Dissektion schalter- oder pedalbetätigt erfolgt, wobei jeweils die entsprechenden elektronischen Kommunikationsmittel zwischen Schalter (der an der Dissektionssonde 20 vorliegt) oder Pedal und den Mess-, Steuerungs- und Regelungsmitteln 17 vorliegen müssen.

Erfindungsgemäß sind nach beiden Weisen die Umschaltmittel von der Umschaltvorrichtung umfasst, die elektronisch mit der Mess-, Steuerungs- und Regelungsvorrichtung 17 gekoppelt oder die in die Mess-, Steuerungs- und Regelungsvorrichtung 17 integriert sind.

Nach der ersten Weise, gezeigt in Fig. 2a, 2b und 3a, wird vorgeschlagen, dass die Umschaltvorrichtung, die es ermöglicht, dass CO₂ entweder aus der Gewebedissektionssonde 20 oder aus der Insufflationskanüle 30 ausgegeben wird, die Gaszuführleitung 2 als eine Y-Gaszuführleitung 2 umfasst. Dabei ist der Abschnitt 23 mit dem ersten Abgang 12 des Insufflators 10 verbunden. Der Abschnitt 23 wird an der Verzweigung 25, siehe auch Detail A in Fig. 2b, in einen ersten distalen Abschnitt 31, der mit der Insufflationskanüle 30 verbunden ist, und einen zweiten distalen Abschnitt 21, der mit der Gewebedissektionssonde 20 verbunden ist, geteilt. Dabei ist vorliegend als Umschaltmittel an der Verzweigung 25 der Y-Gaszuführleitung 2 eine Weiche 22 eingebaut, die durch die Leitung 24' elektronisch mit der Mess-, Steuerungs- und Regelungsvorrichtung 17 verbunden ist und so sicher stellt, dass bei Umschalten der Weiche 22 vom Insufflations- in den Dissektionsmodus oder umgekehrt unmittelbar die entsprechende Signalgebung zur Veränderung der Parameter wie Gas- und CO₂-Druck über die Mess-, Steuerungs- und Regelungsvorrichtung 17 erfolgt. Statt einer Weiche 22 kann hier auch ein Umschaltventil vorliegen, das die Gaszuführung von dem Abschnitt 23 in jeweils einen der distalen Abschnitte 31,21 zulässt.

So wird in einer Insufflationsvorrichtung mit nur einem Insufflator 10 eine Einrichtung zur Aktivierung und Umschaltung von regulärer Gasinsufflation auf einen besonderen Gasinsufflations-Modus, den Dissektionsmodus, aus dem sterilen Bereich heraus zur Gewebedilatation bereitgestellt. Es ergibt sich die Besonderheit, dass kein weiteres Insufflations-Gerät zusätzlich benötigt wird, vielmehr wird beispielsweise (Weise 1) mittels "Y-Weiche 22 mit el. Umschalter" der Gasfluss und die Möglichkeit der besonderen Modus-Aktivierung zusammen in einem Handlungsablauf vom Operateur selbst manuell (oder elektromotorisch) umgestellt, etwa durch den an der Dissektionssonde 20 vorliegenden Schalter.

Das "mechanisch-elektrische Y-Weichen-Stück 22" ist vorliegend in das reguläre Insufflationssystem integriert. Bei dessen Umstellung auf den "Dissektionsmodus" (kommuniziert über die elektrische Leitung 24'), wird der reguläre Insufflationspfad, also der Fluidpfad, über den das Gas zur Erweiterung der Körperhöhle eingeleitet wird, mechanisch verschlossen. Zeitgleich erfolgt über die elektrische Verbindung, in Fig. 3a etwa über die Elektroleitung 24 zur Dissektionskanüle 20 hin, die einen elektrischen Taster bzw. Schalter (nicht dargestellt) zur manuellen und kontrollierten Aktivierung des speziellen Modus integriert bzw. aufgesetzt hat, die Freigabe des CO₂-Flusses über die Dissektionssonde 20 nach dem Dissektionsmodus, also der entsprechenden Parametereinstellung in Bezug auf Gasdruck und Durchfluss. Die Umschaltung und Freigabe des neuen Gasweges erfolgt quasi gleichzeitig. Somit kann nach einer Ausführungsform der erfindungsgemäßen Vorrichtung per Schalteraktivierung an der Dissektionssonde 20 der spezielle Dissektionsmodus aktiviert werden, es kann hierüber aber auch der übliche reguläre Insufflationsvorgang bei "regulärer Y-Weichenstellung" mit der Druck-Flow-Regelung gemäß Insufflationsmodus betrieben werden.

Es kann auch vorgesehen werden, dass die Umschaltvorrichtung jeweils eine mechanisch oder elektronisch betätigbare Gasabsperrvorrichtung in den distalen Abschnitten 31,21 aufweist (nicht gezeigt) die bevorzugt auch mit einer elektronischen Kommunikationsverbindung zur Verbindung mit der Mess-, Steuerungs- und Regelungsvorrichtung 17 ausgestattet ist, um durch das Absperren des distalen Abschnitts 31 der einen Gaszuführleitung 2 den Modus zu aktivieren, der durch den zweiten distalen Abschnitt 21'ausführbar ist.

Die Weiche 22 kann mechanisch oder elektronisch betätigbar ausgeführt sein.

Die zweite Weise, nach der das erfindungsgemäße Prinzip realisiert werden kann, ist in einer Ausführungsform in Fig. 3b skizziert: Zur Gasausgabe entweder aus der Gewebedissektionssonde 20 oder aus der Insufflationskanüle 30 stehen zwei Gaszuführleitungen 21',31' zur Verfügung, die über die Anschlüsse 12 bzw. 12' mit dem Insufflator 10 verbunden sind. Die erste Gaszuführleitung 31' ist einenends mit der Insufflationskanüle 30 und andernends über den zweiten Abgang 12' und einen ersten Fluidpfad 31" mit der Umschalteinheit 16 verbunden, die hier die notwendigen stromaufwärts liegenden Umschaltmittel bildet. Die zweite Gaszuführleitung 21' ist einenends mit der Gewebedissektionssonde 20 und anderenends über einen ersten Abgang 12, der an dem Gehäuse 10 angeordnet ist, und über einen zweiten Fluidpfad 2' mit der Umschalteinheit 16 verbunden. Die Umschalteinheit 16 ist an der Mess-, Steuerungs- und Regelungsvorrichtung 17 angeordnet und kommuniziert mit dieser elektronisch und folglich operativ.

Die Umschalteinheit 16 kann als Weiche oder Umschaltventil ausgebildet sein und bewirkt die Gaszuführung in jeweils einen der Fluidpfade 31 ",2' und von dort weiter in die Leitung 21' oder 31'. Denkbar wäre auch, dass der Umschalteinheit 16 eine jedem der Fluidpfade 31 ", 2' zugeordnete mechanisch oder elektronisch betätigbare Gasabsperrvorrichtung zugeordnet ist, so dass durch einfaches Absperren nur der Weg in die weitere Gaszuführleitung verbleibt und damit eine Umlenkung bewirkt wird. Eine elektronische Verbindung zwischen dem Trokar 30 oder daran vorliegenden Absperr- oder Betätigungsmitteln und der Mess-, Steuerungs- und Regelungsvorrichtung 17 kann über das Elektrokabel 24" oder auch kabellos erfolgen; die entsprechende elektronische Kommunikationsverbindung zwischen der Dissektionssonde 20 oder deren Schalter bzw. ebenfalls Absperr- oder Betätigungsmitteln kann über das Elektrokabel 24 oder auch kabellos ausgeführt sein.

Die Gewebedissektionssonde 20 kann nach beiden Weisen den beschriebenen Schalter aufweisen, der elektronisch direkt oder über die Mess-, Steuerungs- und Regelungsvorrichtung 17 mit den Umschaltmitteln operativ verbunden sein kann, oder sie kann per Pedal od. Taster bzw. elektrisch kapazitivem Tasterfeld ebenfalls elektronisch direkt oder über die Mess-, Steuerungs- und Regelungsvorrichtung 17 aktiviert werden.

Beim Aktivieren kann ein motorisch angetriebenes Stellglied, respektive eine Weiche 22 oder ein Ventil, im Falle der Y-Gaszuführungsleitung den Zugang zur Sonde blockieren und verschließen und zugleich den Weg zur Kanüle 30 für den CO₂-Insufflationsvorgang freigeben. Umgekehrt, wenn der Dissektionsvorgang (Sonde 20) eingeleitet werden soll, funktioniert die Freigabe des Fluidpfades analog.

Wie der Fachmann weiß, ist bei der Aktivierung der CO₂-Dissektion üblicher Weise der CO₂-Druck höher als zuvor bei dem regulären Insufflationsvorgang. Dieser Wert wird üblicher Weise vom Anwender voreingestellt, etwa über ein Bedienfeld, einen Drehregler oder andere geeignete Mittel, und so auch begrenzt.

Bei der Laparoskopie beispielsweise sind i. d. R. 10-15 mmHg vorgesehen.

Für den Dissektionsbetrieb könnte die Einstellung Beispielsweise +5 mmHg - 20mmHg mehr vorsehen gegenüber dem üblicher Weise manuell limitierten Wert. An der Dissektionskanüle 20 werden der Durchfluss und der Druck durch die kleine distale Öffnung begrenzt, je nach Öffnungsgröße resultiert daraus ein höherer od. geringerer Staudruck im Dissektionsweg, jedoch nur ein maximaler Durchfluss und Insufflationsdruck in das Abdomen. Da durch die mechanische Engstelle der Dissektionssonde 20 der Durchfluss immer geringer ist als durch die größere Öffnung des Trokars 30, kann gegebenenfalls die Einstellung der Durchflussrate und die Begrenzung für die Dissektion beibehalten werden, wie sie für die reguläre CO₂-Insufflation bereits bspw. manuell vom Operateur eingestellt ist.

Es wird normalerweise ein recht geringer Durchfluss pro Minute resultieren, was auch eine Sicherheit für den Patienten darstellt, da deshalb das CO₂-Volumen im Abdomen nicht schnell zunehmen kann und so bei dem geschlossenen System lediglich ein sehr langsamer Druckanstieg folgt.

Es sind beispielhaft folgende Insufflationseinstellungen für CO₂ limitiert und vordefiniert:
Pädiatrie: Druck 1-15 mmHg; Flow: 0-15 l/min
Standard: D: 1-30 mmHg; F: 0.50 l/min
Bariatric: D: 1-30 mmHg; F: 0-50 l/min
Gastro: D: 1-30 mmHg; F: 0-5 l/min
Vessel Harvesting: D: 1-15 mmHg; F: 0-10 l/min
Thorakoskopie: D: 0-15 mmHg; F: 0-21/min
Proktoskopie:D: 1-30 mmHg; F: 0-15 l/min.

Im Dissektionsmodus hingegen ist D: +5-20 mmHg gegenüber dem limitierten regulären Wert vorgesehen. Die Erhöhung kann vom Anwender prinzipiell immer vorgegeben werden, bspw. in einem Grundeinstellungsmenü.

Die vorstehend dargelegte zweite Weise wie an Hand von Fig. 3b erläutert, sieht also einen Weg (Fluidpfad) zum Trokar 30 und einen zweiten zur Dissektionskanüle 20 oder -sonde 20 vor. In diesem Falle kann bei Dissektionsinsufflation zeitgleich der dabei entstehende abdominelle Druck über den regulären CO₂-Insufflationsweg gemessen werden, was ein Sicherheitsvorteil ist. In diesem Falle hat daher der Insufflator 10 vorteilhaft die beiden Ausgänge 12,12'. Diese Variante ist kostengünstig, da der Druckregel-Insufflations-Messbereich nur einmal in die Vorrichtung integriert werden muss.

Die max. Aktivierungszeit der Dissektionssonde 20 gemäß beiden Weisen des erfindungsgemäßen Prinzips kann aus Sicherheitsgründen zeitlich begrenzt sein; ebenso der maximal zulässige erhöhte Insufflationsdruck. Hierzu sieht die Mess-, Steuerungs- und Regelungsvorrichtung 17 entsprechende Einstellmöglichkeiten vor. Geeigneter Weise liegt ferner eine Speicher- und Datenverarbeitungsvorrichtung, gekoppelt mit der Mess-, Steuerungs- und Regelungsvorrichtung 17, im Gehäuse des Insufflators 10 vor.

Während der Ausführung des Dissektionsmodus unter Ausübung des erfindungsgemäßen Verfahrens zum alternierenden Insufflieren von Gas in eine Körperhöhlung und pneumatischem Dissezieren in derselben unter Verwendung der erfindungsgemäßen Vorrichtung wird vorgeschlagen, die Überdruckalarmierung zu unterdrücken bzw. auf den erhöhten Wert des Dissektionsmodus anzupassen bzw. in vorstehender Vorrichtung einzustellen, da ein höherer Innendruck im Körper als der reguläre Insufflationsdruck zur Gewebedissektion notwendig ist und zur Dissektionsseite hin abgegeben werden muss. Druckablass von unerwünschtem Überdruck kann durch Ablassen mittels eines weiteren Trokars, der im Insufflationssystem vorgesehen ist, oder auch über einen separaten flexiblen Dissektionsschlauch mit Kanüle erreicht werden und dort über einen zweiten, im flexiblen Instrument befindlichen Kanal kontrolliert abgelassen werden.

Diese Maßnahme reduziert den notwendigen Druck zur Dissektion, da dieser Dissektionsdruck immer in einem geschlossenen System gegen den Innendruck im bspw. Abdomen entgegenwirken muss, um eine Dissektion zu erwirken. Die Ablasseinrichtung ist mit dem Insufflationsgerät ebenfalls elektronisch verbunden und öffnet die Absperreinrichtung immer dann, wenn der Dissektionsmodus ausgelöst wird. Das Öffnen der Absperreinrichtung könnte auch durch Messen des Gas-Überdrucks automatisiert erfolgen.

Die Überprüfung des Gasdrucks in der Körperhöhle kann auch durch eine doppellumige Gasinsufflationszu- und rückführleitung erfolgen (nicht figurativ gezeigt). Dabei dient ein Lumen zur Hinführung des Gases, das andere zur Rückführung zum Insufflator 10 und dort zur Druckmessung und der gezielten geregelten Druckreduzierung bei manueller Aktivierung des Dissektionsmodus. Das Lumen, mittels dessen Gas rückgeführt wird, ist mit einem Gas-Eingang am Insufflator 10 angebunden und bietet die Möglichkeit der permanenten Gasdruck-Überwachung auch im regulären Insufflationsbetrieb. Dies wäre vorteilhaft, da dort keine Pumppausen zur Druckmessung notwendig wären, was die Insufflationszeit und Genauigkeit der Messung erhöht und die Operationszeit vorteilhaft reduziert.

## Patentansprüche

1. Insufflationsvorrichtung, umfassend
einen Insufflator (10), zumindest eine Gaszuführleitung, und zumindest eine Insufflationskanüle (30), die zum Insufflieren eines Insufflations-Gases über die Gaszuführleitung mit dem Insufflator (10) verbunden ist, der ein Gehäuse (11) mit einem Eingang (15) und zumindest einem ersten Abgang (12) für das Insufflations-Gas aufweist, an den die zumindest eine Gaszuführleitung angeschlossen ist, wobei in dem Gehäuse (11) zwischen dem Eingang (15) und dem ersten Abgang (12) ein Fluidpfad vorliegt, in den eine Mess-, Steuerungs- und Regelungsvorrichtung (17) zum gesteuerten und geregelten Ausgeben des Gases zwischengeschaltet ist, die elektronisch mit Bedienmitteln (3) verbunden ist,
- die Insufflationsvorrichtung eine Umschaltvorrichtung aufweist, die dazu ausgebildet ist, eine Umschaltung des geregelten und gesteuerten Gasflusses zur Gasausgabe entweder aus der Gewebedissektionssonde (20) oder aus der zumindest einen Insufflationskanüle (30) bereitzustellen.
**dadurch gekennzeichnet, dass**
- der Insufflator (10) ferner über eine Gaszuführleitung zusätzlich mit einer pneumatischen Gewebedissektionssonde (20) fluidisch verbunden ist, die elektronisch mit der Mess-, Steuerungs- und Regelungsvorrichtung (17) verbunden ist.

2. Insufflationsvorrichtung nach Anspruch 1,
**dadurch gekennzeichnet, dass**
die Umschaltvorrichtung Umschaltmittel umfasst, die elektronisch mit der Mess-, Steuerungs- und Regelungsvorrichtung (17) gekoppelt oder die in die Mess-, Steuerungs- und Regelungsvorrichtung (17) integriert sind.

3. Insufflationsvorrichtung nach Anspruch 1 oder 2,
**dadurch gekennzeichnet, dass**
die Umschaltvorrichtung zur Gasausgabe entweder aus der Gewebedissektionssonde (20) oder aus der zumindest einen Insufflationskanüle (30) umfasst, dass die Gaszuführleitung (2) als eine Y-Gaszuführleitung (2) ausgebildet ist, wobei ein Abschnitt (23) mit dem ersten Abgang (12) verbunden ist und sich der Abschnitt (23) an einer Verzweigung (25) in
- einen ersten distalen Abschnitt (31) erstreckt, der mit der Insufflationskanüle (30) verbunden ist, und
- einen zweiten distalen Abschnitt (21) erstreckt, der mit der Gewebedissektionssonde (20) verbunden ist, und wobei die Umschaltmittel an der Verzweigung (25) der Y-Gaszuführleitung (2) vorgesehen sind, wobei die Umschaltmittel bevorzugt eine Weiche (22) oder ein Umschaltventil sind, die/das die Gaszuführung von dem Abschnitt (23) in jeweils einen der distalen Abschnitte (31,21) zulässt.

4. Insufflationsvorrichtung nach Anspruch 2 oder 3,
**dadurch gekennzeichnet, dass**
die Umschaltvorrichtung zur Gasausgabe entweder aus der Gewebedissektionssonde (20) oder aus der zumindest einen Insufflationskanüle (30) umfasst, dass die Gaszuführleitung (2) als eine Y-Gaszuführleitung (2) ausgebildet ist, wobei ein Abschnitt (23) mit dem ersten Abgang (12) verbunden ist und sich der Abschnitt (23) an einer Verzweigung in
- einen ersten distalen Abschnitt (31) erstreckt, der mit der Insufflationskanüle (30) verbunden ist, und
- einen zweiten distalen Abschnitt (21) erstreckt, der mit der Gewebedissektionssonde (20) verbunden ist, und wobei die Umschaltvorrichtung jeweils eine mechanisch oder elektronisch betätigbare Gasabsperrvorrichtung in den distalen Abschnitten (31,21) aufweist, bevorzugt eine Gasabsperrvorrichtung, die die Gaszuführung von dem Abschnitt (23) in jeweils einen der distalen Abschnitte (31,21) zulässt, und die mit einer elektronischen Kommunikationsverbindung (24) zur Verbindung mit der Mess-, Steuerungs- und Regelungsvorrichtung (17) ausgestattet ist.

5. Insufflationsvorrichtung nach Anspruch 3,
**dadurch gekennzeichnet, dass**
die Weiche (22) oder das Umschaltventil mechanische oder elektronische Betätigungsmittel aufweisen.

6. Insufflationsvorrichtung nach Anspruch 2,
**dadurch gekennzeichnet, dass**
zur Gasausgabe entweder aus der Gewebedissektionssonde (20) oder aus der zumindest einen Insufflationskanüle (30) zwei Gaszuführleitungen (21',31') bereitgestellt sind, wobei
die Umschaltmittel eine Umschalteinheit (16) aufweisen, und wobei
- eine erste Gaszuführleitung (31') einenends mit der Insufflationskanüle (30) und andernends über den zweiten Abgang (12') und einen ersten Fluidpfad (31") mit der Umschalteinheit (16) verbunden ist, und
- eine zweite Gaszuführleitung (21') einenends mit der Gewebedissektionssonde (20) und anderenends über einen ersten Abgang (12), der an dem Gehäuse (10) angeordnet ist, und über einen zweiten Fluidpfad (2') mit der Umschalteinheit (16) verbunden ist.

7. Insufflationsvorrichtung nach Anspruch 6,
**dadurch gekennzeichnet, dass**
die Umschalteinheit (16) zumindest eine Weiche oder ein Umschaltventil zur Gaszuführung in jeweils einen der Fluidpfade (31",2') oder jeweils eine jedem der Fluidpfade (31",2') zugeordnete mechanisch oder elektronisch betätigbare Gasabsperrvorrichtung aufweist.

8. Insufflationsvorrichtung nach zumindest einem der Ansprüche 1 bis 7, **dadurch gekennzeichnet, dass**
die Gewebedissektionssonde (20) einen Schalter aufweist, der elektronisch direkt oder über die Mess-, Steuerungs- und Regelungsvorrichtung (17) mit den Umschaltmitteln operativ verbunden ist.

9. Insufflationsvorrichtung nach zumindest einem der Ansprüche 1 bis 7,
**dadurch gekennzeichnet, dass**
die Insufflationsvorrichtung ein Pedal aufweist, das elektronisch direkt oder über die Mess-, Steuerungs- und Regelungsvorrichtung (17) mit den Umschaltmitteln operativ verbunden ist.

10. Insufflationsvorrichtung nach einem der Ansprüche 1 bis 9,
**dadurch gekennzeichnet, dass**
- die Insufflationskanüle in einem Trokar (30) umfasst ist und/oder
- das Gas CO₂ ist.

11. Insufflationsvorrichtung nach zumindest einem der Ansprüche 1 bis 10,
**dadurch gekennzeichnet, dass**
zumindest eine der Gaszuführleitungen (21',31'), die mit der Insufflationskanüle (30) und/oder mit der pneumatischen Gewebedissektionssonde (20) verbunden ist/sind, doppellumig ausgebildet ist/sind,
wobei ein erstes Lumen zur Zuführung des Gases in die Insufflationskanüle (30) und/oder die Gewebedissektionssonde (20), und ein zweites Lumen zur Rückführung zumindest eines Teils des Gases in die Mess-, Steuerungs- und Regelungsvorrichtung (17) in den Insufflator (10) ausgebildet ist.

12. Insufflationsvorrichtung nach zumindest einem der Ansprüche 1 bis 10,
**dadurch gekennzeichnet, dass**
die Insufflationsvorrichtung zumindest eine Gasrückführleitung umfasst, die nicht die mit der Insufflationskanüle (30) und/oder mit der pneumatischen Gewebedissektionssonde (20) verbundene Gaszuführleitung (2,21',31') ist/sind, und die zur Rückführung zumindest eines Teils des Gases über einen Anschluss in die Mess-, Steuerungs- und Regelungsvorrichtung (17) in den Insufflator (10) ausgebildet ist.

13. Insufflationsvorrichtung nach einem der Ansprüche 8 bis 12,
**dadurch gekennzeichnet, dass**
eine elektronische Verbindung zwischen
- dem Schalter, der an der Gewebedissektionssonde (20) vorliegt, und/oder
- den Gasabsperrvorrichtungen und/oder
- den Umschaltmitteln an der Verzweigung (25) und/oder
- dem Pedal
und der Mess-, Steuerungs- und Regelungsvorrichtung (17) direkt durch einen Anschluss über jeweils eine elektrische Leitung
oder durch jeweils eine Sender-Empfängervorrichtung zur Übermittlung eines elektronischen Signals bereitgestellt wird, oder
indirekt
über eine Kommunikations-BUS-Verbindung, die mit dem Insufflator (10) hergestellt ist.

14. Insufflationsvorrichtung nach zumindest einem der Ansprüche 1 bis 11,
**dadurch gekennzeichnet, dass**
eine Aktivierungsdauer der Gewebedissektionssonde (20) vorbestimmbar und mit einem Timer, der mit der Mess-, Steuerungs- und Regelungsvorrichtung (17) operativ gekoppelt ist, voreinstellbar ist.

15. Insufflationsvorrichtung nach Anspruch 14,
**dadurch gekennzeichnet, dass**
die Insufflationsvorrichtung eine Signalvorrichtung zur Ausgabe eines akustischen und/oder optischen Warnsignals aufweist, die einstellbar ist, ein Warnsignal bei Überschreiten
- eines vorbestimmte Insufflationsdrucks,
- einer vorbestimmten Gasdurchflussrate des Gases, das in die Gaszuführleitung (2,21',31') zugeführt wird, oder
- der Aktivierungsdauer der Gewebedissektionssonde (20) auszugeben.

## Claims

1. Insufflation device, comprising
an insufflator (10), at least one gas supply line and at least one insufflation cannula (30), which is connected to the insufflator (10) for the purposes of insufflating an insufflation gas by way of the gas supply line, said insufflator having a housing (11) with an inlet (15) and at least one first outlet (12) for the insufflation gas, to which the at least one gas supply line is connected, wherein there is a fluid path in the housing (11) between the inlet (15) and the first outlet (12), interposed on which there is a measurement, control and regulation device (17) for release of the gas under control and regulation, said device being electronically connected to operating means (3), the insufflation device has a switchover device embodied to provide a switchover of the gas flow under control and regulation for the purposes of releasing gas either from the tissue dissection probe (20) or from the at least one insufflation cannula (30),
**characterized in that**
- the insufflator (10), by way of a gas supply line, furthermore has an additional fluid connection to a pneumatic tissue dissection probe (20), the latter being connected electronically to the measurement, control and regulation device (17).

2. Insufflation device according to Claim 1, **characterized in that** the switchover device comprises switchover means which are coupled electronically to the measurement, control and regulation device (17) or which are integrated into the measurement, control and regulation device (17).

3. Insufflation device according to Claim 1 or 2,
**characterized in that**
the switchover device, for the purposes of releasing the gas either from the tissue dissection probe (20) or from the at least one insufflation cannula (30), comprises that the gas supply line (2) is embodied as a Y gas supply line (2), wherein one portion (23) is connected to the first outlet (12) and the portion (23), at a junction (25),
- extends into a first distal portion (31) connected to the insufflation cannula (30) and
- extends into a second distal portion (21) connected to the tissue dissection probe (20), and
wherein
the switchover means are provided at the junction (25) of the Y gas supply line (2), wherein the switchover means preferably are a switching unit (22) or a switchover valve which allows the gas supply from the portion (23) into respectively one of the distal portions (31, 21).

4. Insufflation device according to Claim 2 or 3,
**characterized in that**
the switchover device, for the purposes of releasing the gas either from the tissue dissection probe (20) or from the at least one insufflation cannula (30), comprises that the gas supply line (2) is embodied as a Y gas supply line (2), wherein one portion (23) is connected to the first outlet (12) and the portion (23), at a junction,
- extends into a first distal portion (31) connected to the insufflation cannula (30) and
- extends into a second distal portion (21) connected to the tissue dissection probe (20), and
wherein
the switchover device in each case has a mechanically or electronically actuatable gas shut-off device in the distal portions (31, 21), preferably a gas shut-off device which allows the gas supply from the portion (23) into in each case one of the distal portions (31, 21) and which is equipped with an electronic communication connection (24) for the purposes of a connection with the measurement, control and regulation device (17).

5. Insufflation device according to Claim 3,
**characterized in that**
the switching unit (22) or the switchover valve has mechanical or electronic actuation means.

6. Insufflation device according to Claim 2,
**characterized in that**
two gas supply lines (21', 31') are provided for releasing gas either from the tissue dissection probe (20) or from the at least one insufflation cannula (30), wherein
the switchover means have a switchover unit (16) and wherein
- a first gas supply line (31') is connected at one end to the insufflation cannula (30) and at the other end to the switchover unit (16) by way of the second outlet (12') and a first fluid path (31 "), and
- a second gas supply line (21') is connected at one end to the tissue dissection probe (20) and at the other end to the switchover unit (16) by way of a first outlet (12), which is arranged at the housing (10), and by way of a second fluid path (2').

7. Insufflation device according to Claim 6,
**characterized in that**
the switchover unit (16) has at least one switching unit or one switchover valve for guiding gas in one of the fluid paths (31", 2') in each case or has, in each case, a mechanically or electronically actuatable gas shut-off device assigned to each one of the fluid paths (31 ", 2').

8. Insufflation device according to at least one of Claims 1 to 7, **characterized in that** the tissue dissection probe (20) has a switch which is operatively connected to the switchover means, either directly electronically or by way of the measurement, control and regulation device (17).

9. Insufflation device according to at least one of Claims 1 to 7,
**characterized in that**
the insufflation device has a pedal which is operatively connected to the switchover means, either directly electronically or by way of the measurement, control and regulation device (17).

10. Insufflation device according to one of Claims 1 to 9,
**characterized in that**
- the insufflation cannula is comprised in a trocar (30) and/or
- the gas is CO₂.

11. Insufflation device according to at least one of Claims 1 to 10,
**characterized in that**
at least one of the gas supply lines (21', 31'), which is/are connected to the insufflation cannula (30) and/or the pneumatic tissue dissection probe (20), has/have an embodiment with two lumens,
wherein a first lumen is embodied to supply the gas into the insufflation cannula (30) and/or the tissue dissection probe (20) and a second lumen is embodied to return at least some of the gas into the measurement, control and regulation device (17) in the insufflator (10).

12. Insufflation device according to at least one of Claims 1 to 10,
**characterized in that**
the insufflation device comprises at least one gas return line which is not the gas supply line (2, 21', 31') connected to the insufflation cannula (30) and/or the pneumatic tissue dissection probe (20), and which is embodied to return at least some of the gas by way of a connector into the measurement, control and regulation device (17) in the insufflator (10).

13. Insufflation device according to one of Claims 8 to 12,
**characterized in that**
an electronic connection is provided between
- the switch, which is available at the tissue dissection probe (20), and/or
- the gas shut-off devices and/or
- the switchover means at the junction (25) and/or
- the pedal
and the measurement, control and regulation device (17), directly by a connector by way of in each case one electric line
or by in each case a transmitter-receiver device for transmitting an electronic signal, or indirectly
by way of a communication BUS connection, which is established with the insufflator (10).

14. Insufflation device according to at least one of Claims 1 to 11,
**characterized in that**
an activation duration of the tissue dissection probe (20) is predeterminable and settable in advance by way of a timer which is operatively coupled to the measurement, control and regulation device (17).

15. Insufflation device according to Claim 14,
**characterized in that**
the insufflation device has a signal device for emitting an acoustic and/or optical warning signal, said signal device being settable to emit a warning signal in the case of
- a predetermined insufflation pressure,
- a predetermined gas flow rate of the gas, which is supplied in the gas supply line (2, 21', 31')
or
- the activation duration of the tissue dissection probe (20)
being exceeded.

## Revendications

1. Dispositif d'insufflation comprenant :
un insufflateur (10), au moins une conduite d'acheminement de gaz et au moins une canule d'insufflation (30) qui sont reliées à l'insufflateur (10) pour insuffler un gaz d'insufflation par l'intermédiaire de la conduite d'acheminement de gaz, lequel insufflateur comprend un boîtier (11) muni d'un orifice d'entrée (15) et d'au moins un premier orifice de sortie (12) destiné au gaz d'insufflation, auquel est raccordée l'au moins une conduite d'acheminement de gaz, dans lequel il est prévu dans le boîtier (11) entre l'orifice d'entrée (15) et le premier orifice de sortie (12) un trajet de fluide dans lequel est interposé un dispositif de mesure, de commande et de régulation (17), destiné à commander et réguler la fourniture en sortie du gaz, qui est électroniquement connecté à des moyens d'actionnement (3),
- le dispositif d'insufflation comprend un dispositif de basculement qui est conçu pour effectuer un basculement du flux de gaz régulé et commandé vers l'orifice de sortie soit à partir de la sonde de dissection de tissu (20), soit à partir de l'au moins une canule d'insufflation (30),
**caractérisé en ce que**
- l'insufflateur (10) est par ailleurs en outre relié de manière fluidique par l'intermédiaire d'une conduite d'acheminement de gaz à une sonde de dissection de tissu pneumatique (20) qui est électroniquement connectée au dispositif de mesure, de commande et de régulation (17).

2. Dispositif d'insufflation selon la revendication 1,
**caractérisé en ce que** le dispositif de basculement comprend des moyens de basculement qui sont électroniquement couplés au dispositif de mesure, de commande et de régulation (17) ou qui sont intégrés au dispositif de mesure, de commande et de régulation (17).

3. Dispositif d'insufflation selon la revendication 1 ou 2,
**caractérisé en ce que** le dispositif de basculement est conçu pour délivrer en sortie un gaz soit à partir de la sonde de dissection de tissu (20), soit à partir de l'au moins une canule d'insufflation (30), de manière à ce que la conduite d'acheminement de gaz (2) soit réalisée sous forme d'une conduite d'acheminement de gaz en Y (2), dans lequel une partie (23) est reliée au premier orifice de sortie (12) et la partie (23) s'étend, au niveau d'une ramification (25),
- dans une première partie distale (31) qui est reliée à la canule d'insufflation (30), et
- dans une seconde partie distale (21) qui est reliée à la sonde de dissection de tissu (20), et dans lequel les moyens de basculement sont prévus au niveau de la ramification (25) de la conduite d'acheminement de gaz en Y (2), dans lequel les moyens de basculement sont de préférence un pointeau (22) ou une soupape de basculement qui laisse passer l'acheminement de gaz depuis la partie (23) vers chacune des parties distales (31, 21).

4. Dispositif d'insufflation selon la revendication 2 ou 3,
**caractérisé en ce que** le dispositif de basculement est conçu pour délivrer en sortie un gaz soit à partir de la sonde de dissection de tissu (20), soit à partir de ladite au moins une canule d'insufflation (30), de manière à ce que la conduite d'acheminement de gaz (2) soit réalisée sous la forme d'une conduite d'acheminement de gaz en Y (2), dans lequel une partie (23) est reliée au premier orifice de sortie (12) et la partie (23) s'étend, au niveau d'une bifurcation,
- dans une première partie distale (31) qui est reliée à la canule d'insufflation (30), et
- dans une seconde partie distale (21) qui est reliée à la sonde de dissection de tissu (20), et dans lequel
le dispositif de basculement comprend respectivement un dispositif d'arrêt des gaz pouvant être actionné mécaniquement ou électroniquement dans les parties distales (31, 21), de préférence un dispositif d'arrêt des gaz qui laisse passer l'acheminement de gaz de la partie (23) vers l'une, respective, des parties distales (31, 21) et qui est conçu pour être connecté au moyen d'une liaison de communication électronique (24) au dispositif de mesure, de commande et de régulation (17).

5. Dispositif d'insufflation selon la revendication 3,
**caractérisé en ce que** le pointeau (22) ou la soupape de basculement comprend des moyens d'actionnement mécaniques ou électroniques.

6. Dispositif d'insufflation selon la revendication 2,
**caractérisé en ce que** deux conduites d'acheminement de gaz (21', 31') sont prévues pour délivrer en sortie un gaz soit à partir de la sonde de dissection de tissu (20), soit à partir de l'au moins une canule d'insufflation (30), dans lequel
les moyens de basculement comprennent une unité de basculement (16), et dans lequel
- une première conduite d'acheminement de gaz (31') est reliée par une extrémité à la canule d'insufflation (30) et par l'autre extrémité, par l'intermédiaire du second orifice de sortie (12') et d'un premier trajet de fluide (31"), à l'unité de basculement (16), et
- une seconde conduite d'acheminement de gaz (21') est reliée par une extrémité à la sonde de dissection de tissu (20) et par l'autre extrémité, par l'intermédiaire d'un premier orifice de sortie (12) qui est disposé sur le boîtier (10) et par l'intermédiaire d'un second trajet de fluide (2'), à l'unité de basculement (16).

7. Dispositif d'insufflation selon la revendication 6,
**caractérisé en ce que** l'unité de basculement (16) comprend au moins un pointeau ou une soupape de basculement destiné à l'acheminement de gaz dans l'un, respectif, des trajets de fluide (31", 2') ou par l'intermédiaire d'un dispositif d'arrêt des gaz pouvant être actionné mécaniquement ou électroniquement respectivement associé à chacun des trajets de fluide (31" 2').

8. Dispositif d'insufflation selon au moins l'une des revendications 1 à 7, **caractérisé en ce que** la sonde de dissection de tissu (20) comprend un commutateur qui est électroniquement connecté de manière directe ou par l'intermédiaire du dispositif de mesure, de commande et de régulation (17) aux moyens de basculement.

9. Dispositif d'insufflation selon au moins l'une des revendications 1 à 7, **caractérisé en ce que** le dispositif d'insufflation comprend une pédale qui est fonctionnellement reliée de manière directe ou par l'intermédiaire du dispositif de mesure, de commande et de régulation (17) aux moyens de basculement.

10. Dispositif d'insufflation selon au moins l'une des revendications 1 à 9, **caractérisé en ce que**
- la canule d'insufflation est contenue dans un trocart (30) et/ou
- le gaz est du CO₂.

11. Dispositif d'insufflation selon au moins l'une des revendications 1 à 10, **caractérisé en ce qu'**au moins l'une des conduites d'acheminement de gaz (21', 31') qui est/sont reliée(s) à la canule d'insufflation (30) et/ou à la sonde de dissection de tissu pneumatique (20), est/sont du type à double lumière,
dans lequel une première lumière est conçue pour l'acheminement du gaz dans la canule d'insufflation (30) et/ou dans la sonde de dissection de tissu (20), et une seconde lumière est conçue pour renvoyer au moins une partie du gaz vers le dispositif de mesure, de commande et de régulation (17) dans l'insufflateur (10).

12. Dispositif d'insufflation selon au moins l'une des revendications 1 à 10, **caractérisé en ce que** le dispositif d'insufflation comprend au moins une conduite de renvoi de gaz qui n'est/ne sont pas la conduite d'acheminement de gaz (2, 21', 31') reliée(s) à la canule d'insufflation (30) et/ou à la sonde de dissection de tissu pneumatique (20), et qui est conçue pour renvoyer au moins une partie du gaz par l'intermédiaire d'un raccordement vers le dispositif de mesure, de commande et de régulation (17) dans l'insufflateur (10).

13. Dispositif d'insufflation selon l'une quelconque des revendications 8 à 12, **caractérisé en ce qu'**il est prévu une liaison électronique entre
- le commutateur qui se trouve sur la sonde de dissection de tissu (20), et/ou
- les dispositifs d'arrêt des gaz et/ou
- les moyens de basculement, sur la ramification (25), et/ou
- la pédale,
et **en ce que** le dispositif de mesure, de commande et de régulation (17) est directement fourni par un raccordement par l'intermédiaire d'une ligne électrique respective,
ou par l'intermédiaire d'un dispositif émetteur-récepteur respectif pour transmettre un signal électronique, ou
de manière indirecte, par l'intermédiaire d'une liaison par bus de communication réalisée avec l'insufflateur (10).

14. Dispositif d'insufflation selon au moins l'une quelconque des revendications 1 à 11,
**caractérisé en ce qu'**une durée d'activation de la sonde de dissection de tissu (20) peut être déterminée à l'avance et peut être réglée à l'avance au moyen d'un temporisateur qui est fonctionnellement couplé au dispositif de mesure, de commande et de régulation (17).

15. Dispositif d'insufflation selon la revendication 14,
**caractérisé en ce que** le dispositif d'insufflation comprend un dispositif de signalisation destiné à délivrer un signal d'alarme acoustique et/ou optique, qui est réglable, afin de délivrer un signal d'alarme lors du dépassement
- d'une pression d'insufflation prédéterminée,
- d'un débit de gaz prédéterminé du gaz qui est acheminé dans la conduite d'acheminement de gaz (2, 21', 31'), ou
- de la durée d'activation de la sonde de dissection de tissu (20).
